# EUROPEAN PATENT APPLICATION

(11) **EP 4 620 641 A1**
(43) Date of publication of application: **24.09.2025**
(21) Application number: 25161361.8
(22) Date of filing: 03.03.2025
(51) Int. Cl.: B26D 1/40, B65H 35/08, A61F 13/15, B65H 39/14

(54) **AN APPARATUS FOR CUTTING AND APPLYING ELASTICALLY STRETCHED TAPE SEGMENTS**

(30) Priority: 22.03.2024 IT 202400006436
(71) Applicant: Fameccanica.Data S.p.A., 66020 San Giovanni Teatino (CH) (IT)
(72) Inventor: SABLONE, Gabriele, 66020 San Giovanni Teatino (Chieti) (IT)
(74) Representative: Buzzi, Notaro & Antonielli d'Oulx S.p.A.

(57) **Abstract**

An apparatus for cutting and applying elastically stretched tape segments, comprising:
- a transfer unit (22) comprising a plurality of transfer elements (24), each of which comprises two gripping elements (28) movable between a close position and a distant position,
- a plurality of anvil elements (30) carried by the transfer unit (22) and moving together with the transfer elements (24), and
- a rotary knife (32) cooperating with the anvil elements (30) to transversely cut successive end portions of a continuous elastic tape (14) held by the transfer elements (24).

## Description

### Field of the Invention

The present invention relates to an apparatus for cutting and applying elastically stretched tape segments.

The invention was developed particularly for application in machines for producing absorbent sanitary articles.

In the following description, reference will be made to this field of application without, however, losing generality.

The present invention also relates to a machine for producing absorbent sanitary articles comprising an apparatus for cutting and applying elastically stretched tape segments and a method for cutting and applying elastically stretched tape segments.

### Prior Art

In machines for producing absorbent sanitary articles, it is often necessary to produce tape segments from a continuous elastic laminate advancing in a longitudinal direction. These tape segments are elastically stretched and are transferred to an application area where they are applied in an elastically stretched state to a composite web precursor of absorbent sanitary articles.

Elastically stretched tape segments are used, for example, to form gasketing elements applied in the waistbands of absorbent sanitary articles to create pockets capable of receiving and containing liquid or semi-liquid fecal material.

For example, US 2020/0038256 A1 describes an absorbent sanitary article including a chassis and at least one gasketing element applied to the topsheet and having an outer transverse edge attached to the topsheet and an inner transverse edge detached from the topsheet so that, during use, the containment element forms a pocket facing the crotch section of the chassis and can receive and contain fecal material.

To form and apply elastically stretched elements, a process can be used that includes:
- feeding a continuous elastic laminate elastically extendable in a transverse direction,
- applying glue to a surface of the continuous elastic laminate,
- transversely cutting the continuous elastic laminate to form tape segments having one side to which glue is applied,
- elastically stretching the tape segments in the transverse direction,
- applying the elastically stretched tape segments transversely to a continuous moving web, and
- securing the elastically stretched tape segments transversely to the moving continuous web via the glue applied to one side of the tape segments.

EP2260813-B1 describes an apparatus and a method for cutting and applying elastic segments under tension to absorbent sanitary articles, in which a continuous elastic tape is transversely cut in a cut-and-slip unit to form an array of elastic segments that are picked up by a spreading device including two wheels rotating around mutually inclined axes, which stretch the elastic segments in the transverse direction.

US 2002/023723 A1 illustrates in figure 27 a transfer assembly for transversely stretching elastic segments, comprising a plurality of transfer elements moving along a closed path, each of which includes two gripping elements configured to grip opposite ends of a respective tape segment and move rectilinearly in a transverse direction between a close position and a distant position so that the gripping elements stretch the respective tape segments transversely during transfer to an application station.

These known solutions have a cutting unit for forming elastic segments that is separate from the transfer unit that performs the transverse stretching of the elastic segments. Therefore, it is necessary to transfer the elastic segments from the cutting unit to the stretching unit, which can result in positioning errors and risks of malfunctions.

In the solutions according to the prior art, the transfer of tape segments from the cutting station to the stretching unit is performed via an anvil wheel on which the elastic segments are cut. This solution makes it difficult to glue the elastic segments to the absorbent sanitary articles because it is not possible to apply the glue to the continuous tape before cutting.

### Object and Summary of the Invention

The object of the present invention is to provide an apparatus for cutting and applying elastically stretched tape segments that overcomes the problems of the prior art.

According to the present invention, this object is achieved by an apparatus having the features defined in claim 1.

According to another aspect, the present invention relates to a machine for producing absorbent sanitary articles as defined in claim 10 and a method for cutting and applying elastically stretched tape segments as defined in claim 9.

The claims are an integral part of the teaching provided in relation to the invention.

### Brief Description of the Drawings

The present invention will now be described in detail with reference to the attached drawings, provided purely by way of non-limiting example, in which:
- Figure 1 is a perspective view of an apparatus according to the present invention,
- Figure 2 is a schematic side view illustrating the operation of an apparatus according to the present invention,
- Figure 3 is an exploded perspective view of the apparatus in Figure 1,
- Figure 4 is a schematic perspective view of the transfer unit indicated by arrow 4 in Figure 1, and
- Figure 5 is a schematic cross-section taken along line V-V in Figure 4.

### Detailed Description

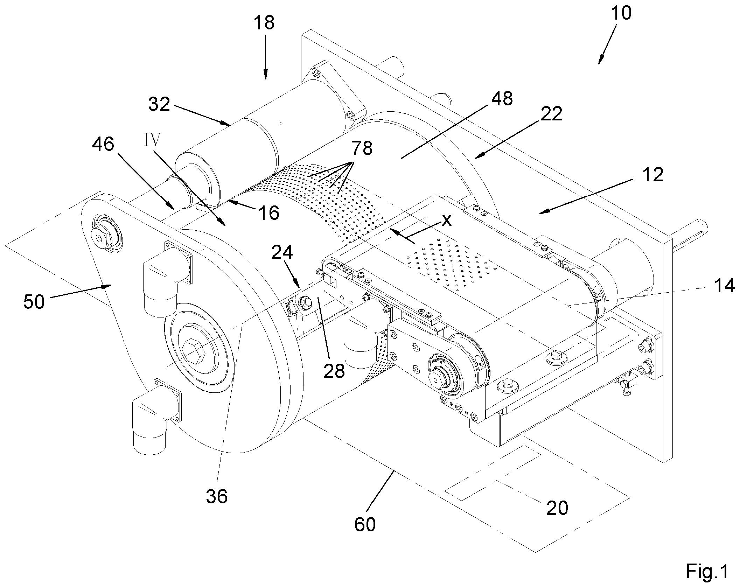

With reference to Figures 1-3, 10 indicates an apparatus for cutting and applying elastically stretched tape segments.

The apparatus 10 includes a feeding device 12 configured to feed a continuous elastic tape 14 in a longitudinal direction X towards a cutting station 16.

The apparatus 10 includes a cutting unit 18 configured to transversely cut the continuous elastic tape 14 at the cutting station 16 to form an array of tape segments 20.

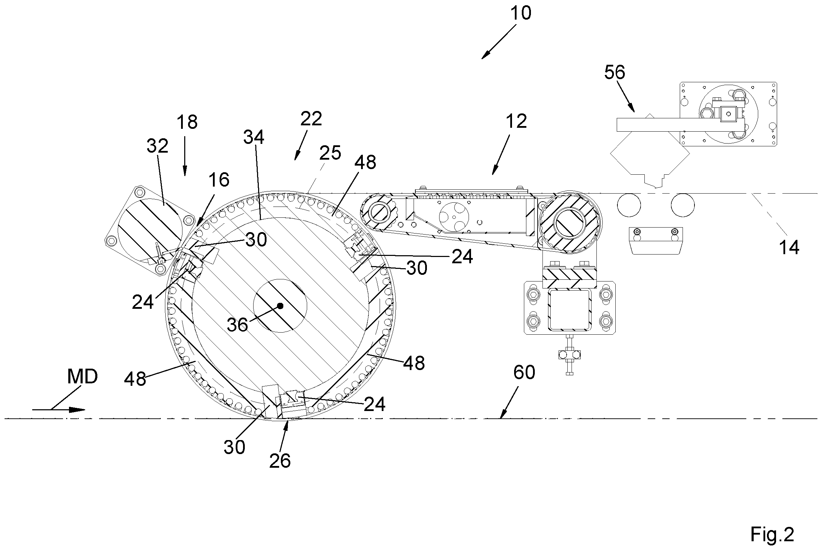

The apparatus 10 includes a transfer unit 22 comprising a plurality of transfer elements 24 movable along a closed path 25 (Figure 2).

The transfer elements 24 are configured to pick up respective tape segments 20 at the cutting station 16 and release them at an application station 26 (Figure 2). At the application station 26, the tape segments 20 are applied in spaced positions on a composite web 60 moving in the machine direction MD, which is a precursor of absorbent sanitary articles.

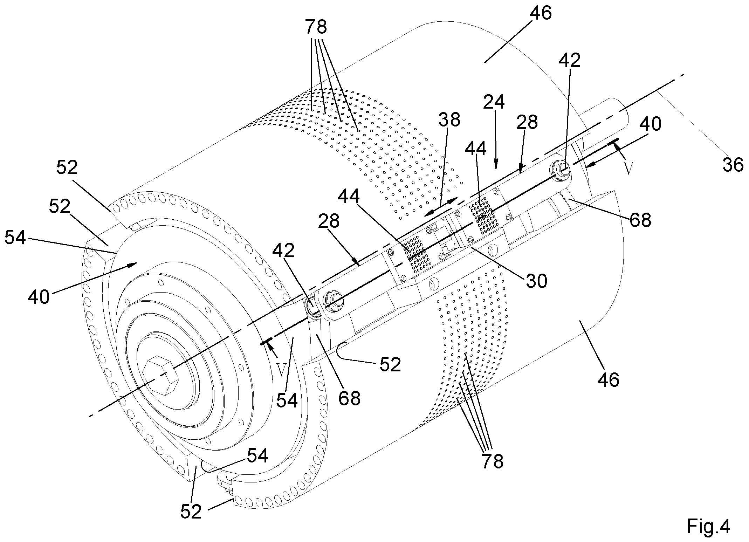

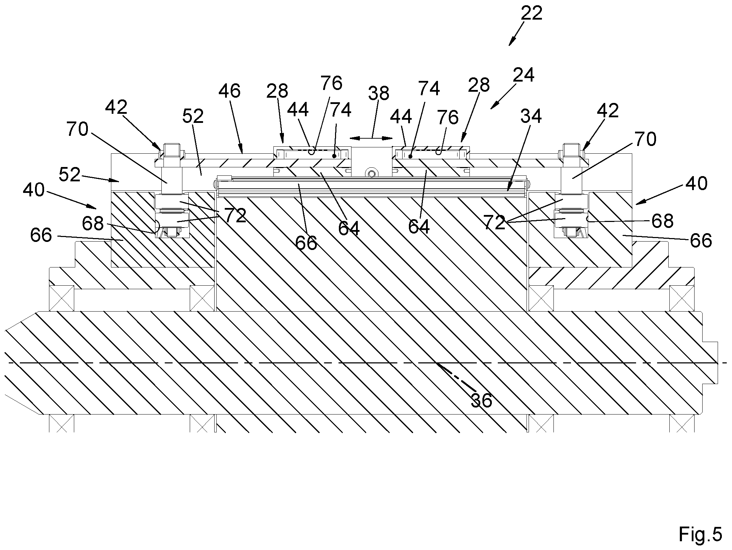

With reference to Figures 4 and 5, each of the transfer elements 24 includes two gripping elements 28 configured to grip opposite ends of a respective tape segment 20. The two gripping elements 28 of each transfer element 24 are movable in a transverse rectilinear direction 38. The two gripping elements 28 are in a close position at the cutting station 16 and in a distant position at the application station 26.

In this way, the gripping elements 28 elastically stretch the respective tape segments 20 in the transverse rectilinear direction 38 during the transfer from the cutting station 16 to the application station 26.

With reference to Figures 2-4, the cutting unit 18 includes a plurality of anvil elements 30 carried by the transfer unit 22. The anvil elements 30 are adjacent to respective transfer elements 24 and move together with the transfer elements 24 along the closed path 25 (Figure 2).

With reference to Figures 1-3, the cutting unit 18 includes a rotary knife 32 that cooperates with the anvil elements 30 to transversely cut successive end portions of the continuous elastic tape 14 at the cutting station 16.

The successive end portions of the continuous elastic tape 14 are held by the transfer elements 24 at the time of cutting.

After the transverse cutting of the end portions of the continuous elastic tape 14 performed at the cutting station 16, successive tape segments 20 are formed, held by the respective transfer elements 24.

The tape segments 20 formed at the cutting station 16 are transferred by the transfer elements 24 from the cutting station 16 to the application station 26. During the transfer from the cutting station 16 to the application station 26, the gripping elements 28 elastically stretch the tape segments 20, and at the application station 26, the tape segments 20 are applied in an elastically stretched state in the transverse direction onto the composite web 60 in spaced positions along the machine direction MD.

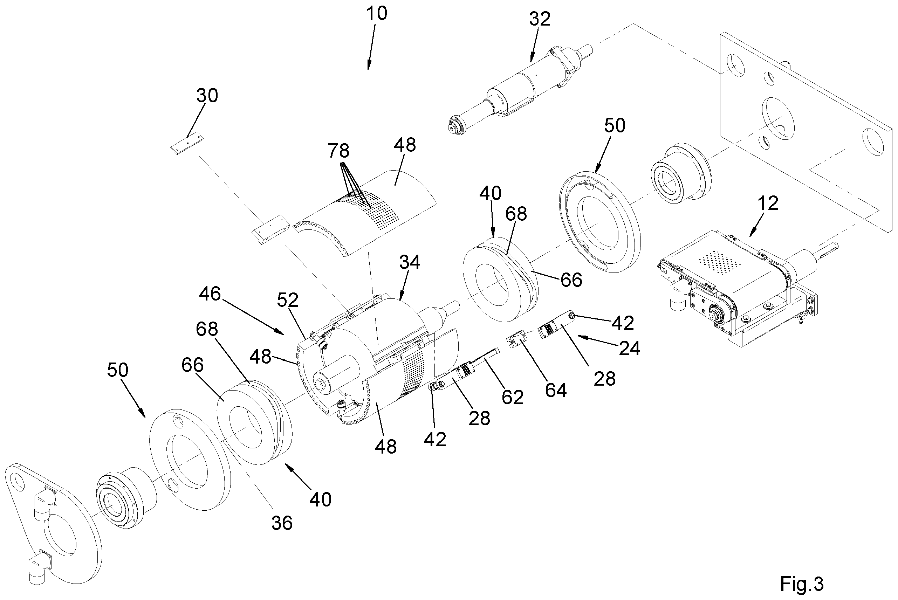

With reference to Figures 3-5, the transfer unit 22 includes a central body 34 rotatable around a transverse rotation axis 36.

The anvil elements 30 are fixed to the central body 34 in angularly spaced positions. For example, the transfer unit 22 may include three anvil elements 30 spaced angularly by 120°.

The gripping elements 28 of each transfer element 24 are rotationally fixed with respect to the central body 34 and are movable relative to the central body 34 between a close position and a distant position along a respective transverse rectilinear direction 38 parallel to the transverse rotation axis 36.

With reference to Figure 5, each transfer element 24 may include a transverse guide 62 fixed to the central body 34, and the two gripping elements 28 may be fixed to respective shoes 64, for example, ball bearing shoes, which slidingly engage the transverse guide 62.

With reference to Figures 4 and 5, the transfer unit 22 includes two stationary cams 40 that control the movement of the gripping elements 28 along their respective transverse rectilinear direction 38.

The stationary cams 40 are located on opposite sides of the central body 34 and cooperate with respective cam followers 42 carried by the gripping elements 28. Each stationary cam 40 may include a disc 66 having a groove 68 engaged by the cam followers 42. Each cam follower 42 may include a pin 70 fixed to the respective gripping element 28 and on which rolling bearings 72 are mounted, engaging the respective groove 68.

In an advantageous embodiment, the transfer unit 22 may include electric drive means that control the movement of the gripping elements 28 along their respective transverse rectilinear direction 38.

With reference to Figures 4 and 5, the gripping elements 28 have respective gripping surfaces 44 configured to grip the ends of the tape segments 20. The gripping surfaces 44 are provided with suction holes connected to a suction system 46 comprising a plurality of suction shells 48 fixed with respect to the central body 34. The suction shells 48 are hollow and provide pneumatic communication between the gripping surfaces 44 of the gripping elements 28 and a stationary suction manifold 50 (Figures 1 and 3). The gripping surfaces 44 may also include protruding teeth designed to enhance the grip on the tape segments 20.

With reference to Figures 3-5, the suction shells 48 have respective front surfaces 52 facing and spaced from corresponding front surfaces 52 of adjacent suction shells 48.

The mutually facing front surfaces 52 of the suction shells 48 form transverse channels 54 between pairs of adjacent suction shells 48. Each transfer element 24 is housed in a respective transverse channel 54.

The anvil elements 30 are housed in the transverse channels 54, where the respective transfer elements 24 are also housed.

The front surfaces 52 of the suction shells 48 are in contact with the side walls of the gripping elements 28 and have suction holes 74 (Figure 5) facing slots 76 formed on the side walls of the gripping elements 28 and communicating with the suction holes on the gripping surfaces 44, establishing pneumatic communication between the gripping surfaces 44 and the suction system 46.

The suction shells 48 have respective cylindrical outer surfaces, on which suction holes 78 can be provided to hold the terminal portion of the continuous elastic tape 14 by suction near the anvil elements 30. In this way, the terminal portion of the continuous elastic tape 14 is held by suction on opposite sides of the anvil elements 30 by the transfer elements 24 and the suction shells 48.

With reference to Figure 2, the apparatus 10 may include a glue applicator device 56 configured to apply a glue pattern to the continuous elastic tape 14 upstream of the cutting station 16, so that the tape segments 20 can be glued to the composite web 60 at the application station 26.

In an advantageous embodiment, the glue pattern applied to the continuous elastic tape 14 may have a C-shape comprising two glue segments arranged longitudinally at opposite ends of a glue segment arranged transversely.

The apparatus 10 according to the present invention can be integrated into a machine for producing absorbent sanitary articles. The apparatus 10 can be used specifically to produce elastic containment elements applied to the waistbands of absorbent sanitary articles to form pockets for collecting and containing liquid or semi-liquid fecal material.

The apparatus 10 according to the present invention constitutes an integrated unit for cutting, stretching, and applying elastic segments. A particularly advantageous feature of the apparatus according to the present invention is that the cutting, stretching, and application operations are performed while the elastic segments are held on the same transfer element. The elastic segments are not transferred from a cutting unit to a transfer unit. This makes the apparatus simpler and more compact, as a separate cutting unit is not required, and the elastic segments do not need to be moved from the cutting unit to the transfer unit. The fact that the elastic segments are cut while held on their respective transfer elements improves the precision with which the elastic segments are applied and reduces the risk of malfunctions.

With the solution according to the present invention, it is possible to apply glue to the continuous elastic tape 14 upstream of the cutting station 16 because, after cutting, the surface with the applied glue does not come into contact with other elements of the apparatus 10.

Of course, without prejudice to the principle of the invention, the construction details and embodiments may vary widely from what has been described and illustrated, without departing from the scope of the invention as defined by the following claims.

## Claims

1. An apparatus for cutting and applying elastically stretched tape segments, comprising:
- a feeding device (12) configured to feed a continuous elastic tape (14) in a longitudinal direction (X) towards a cutting station (16),
- a cutting unit (18) configured to transversely cut said continuous elastic tape (14) at said cutting station (16) to form an array of tape segments (20),
- a transfer unit (22) comprising a plurality of transfer elements (24) movable along a closed path (25) and configured to pick up respective tape segments (20) at the cutting station (16) and release them at an application station (26), wherein each of said transfer elements (24) comprises two gripping elements (28) configured to grip opposite ends of a respective tape segment (20) and movable in a transverse rectilinear direction (38) between a close position at said cutting station (16) and a distant position at said application station (26), such that said gripping elements (28) elastically stretch the respective tape segments (20) in said transverse rectilinear direction (38) during the transfer from the cutting station (16) to the application station (26),
**characterized in that** said cutting unit (18) comprises:
- a plurality of anvil elements (30) carried by said transfer unit (22), adjacent to respective transfer elements (24) and moving together with said transfer elements (24) along said closed path (25), and
- a rotary knife (32) cooperating with said anvil elements (30) to transversely cut successive end portions of said continuous elastic tape (14) held by said transfer elements (24) at said cutting station (16), and **in that** the apparatus comprises a glue applicator device (56) configured to apply a glue pattern to said continuous elastic tape (14) upstream of said cutting station (16).

2. An apparatus according to claim 1, wherein said transfer unit (22) comprises a central body (34) rotatable around a transverse rotation axis (36).

3. An apparatus according to claim 2, wherein the two gripping elements (28) of each transfer element (24) are rotationally fixed with respect to said central body (34) and are movable relative to said central body (34) between a close position and a distant position along a respective transverse rectilinear direction (38) parallel to said transverse rotation axis (36).

4. An apparatus according to claim 2 or claim 3, wherein said anvil elements (30) are fixed to said central body (34).

5. An apparatus according to any of claims 2-4, wherein said transfer unit (22) comprises two stationary cams (40) located on opposite sides of said central body (34) and cooperating with cam followers (42) carried by said gripping elements (28).

6. An apparatus according to any of the preceding claims, wherein said gripping elements (28) have respective gripping surfaces (44) connected to a suction system (46) comprising a plurality of suction shells (48) fixed relative to said central body (34) and in pneumatic communication with the gripping surfaces (44) of said gripping elements (28) and with a stationary suction manifold (50).

7. An apparatus according to claim 6, wherein each of said suction shells (48) has front surfaces (52) facing and spaced from corresponding front surfaces (52) of adjacent suction shells (48) to form a transverse channel (54) between each pair of adjacent suction shells (48), and wherein each transfer element (24) is housed in a respective transverse channel (54).

8. An apparatus according to claim 7, wherein said anvil elements (30) are housed in the transverse channels (54) in which the respective transfer elements (24) are housed.

9. A method for cutting and applying elastically stretched tape segments implemented using an apparatus according to any of the preceding claims.

10. A machine for producing absorbent sanitary articles comprising an apparatus for cutting and applying elastically stretched tape segments according to any of the preceding claims.
